# EUROPÄISCHE PATENTSCHRIFT

(11) **EP 0 868 907 B1**
(45) Veröffentlichungstag und Bekanntmachung des Hinweises auf die Patenterteilung: **15.09.2004**
(21) Anmeldenummer: 98104676.6
(22) Anmeldetag: 16.03.1998
(51) Int. Cl.: A61K 7/48

(54) **Bestandteil eines Hautpflegemittels**
Constitutive part of a skin-care product
Partie constitutive d'un produit pour le soin de la peau

(30) Priorität: 21.03.1997 DE 19711777
(43) Veröffentlichungstag der Anmeldung: 07.10.1998
(73) Patentinhaber: K.D. Pharma Bexbach GmbH, 66450 Bexbach (DE)
(72) Erfinder: Krumbholz, Rudolf, Dr., 57510 Holving (FR); Lembke, Peter, Dr., 43008 Tarragona (ES)
(74) Vertreter: Bernhardt, Winfrid, Dr.-Ing.

(56) Entgegenhaltungen:
- EP-A- 0 271 747
- DE-A- 1 917 088

## Beschreibung

Die Erfindung betrifft einen Harnstoff enthaltenden Bestandteil eines Hautpflegemittels.

Harnstoff ist in Cremes und Salben vielfach anzutreffen als Feuchthaltemittel für die Haut.

Der Erfindung liegt die Aufgabe zugrunde, den Harnstoff in einem weitergehenden Umfang zur Hautpflege nutzbar zu machen.

Gemäß der Erfindung ist zu diesem Zweck vorgesehen, daß der Harnstoff Einschlußverbindungen mit mehrfach ungesättigten Fettsäuren und/oder Verbindungen mehrfach ungesättigter Fettsäuren bildet.

Auf diese Weise werden in dem Hautpflegemittel für die Hautpflege wirksame Fette zur Verfügung gestellt, die gegen Oxidation geschützt sind.

Der Schutz von in das Harnstoffgitter eingebauten Verbindungen vor Luftsauerstoff ist an sich bekannt, z.B. aus RÖMPP CHEMIE LEXIKON", 9. Auflage, Seite 3085. Dort ist angegeben, daß der Harnstoff geradkettige Kohlenwasserstoffe und schwach verzweigte Aliphaten jeweils in einem Kanal seines, in diesem Fall hexagonalen, Gitters einschließen kann: n-Oktan werde eingebaut, Isooktan nicht. Ein Gemisch aus Alkanen lasse sich mittels Harnstoff fraktionieren. Technische Anwendung finde die Bildung von Einschlußverbindungen des Harnstoffs bei der Trennung von Kohlenwasserstoff-Gemischen, bei der Entparaffinierung von Erdöl sowie für Katalysatoren u.a.. Im größeren Gitter des Thioharnstoffs können auch verzweigte und zyklische Aliphaten eingelagert werden.

Eine damit übereinstimmende Information findet sich in der DE 19 17 088 A. In dieser ist eine Badeölzusammensetzung vorgeschlagen, die Einschlussverbindungen von Harnstoff mit besonderen Ölen, insbesondere Weichmacherölen, enthält. Dazu ist ausdrücklich der Hinweis gegeben, die Öle sollen im wesentlichen eine geradkettige Konfiguration im Molekül aufweisen; für Öle mit überwiegend verzweigten Ketten oder zyklische Stukturen solle Thioharnstoff verwendet werden.

Auch der EP 0 271 747 A ist zu entnehmen, dass im wesentlichen nur Fettsäuren mit geradkettigen Molekülen Einschlussverbindungen mit Harnstoff bilden. Es ist ein Verfahren zum Fraktionieren einer mehrfach ungesättigte Fettsäuren enthaltenden Fettsäuremischung beschrieben, bei dem die Fettsäuremischung mit Harnstoff zur Reaktion gebracht und eine Dispersion aus einem festen Einschluß-Komplex und einer flüssigen Phase gebildet wird, aus der man nach Trennung von dem Einschluß-Komplex eine mit den mehrfach ungesättigten Fettsäuren angereicherte Fraktion erhält. Die Trennung beruht darauf, dass die geradkettigen oder weitgehend geradkettigen Moleküle in den Harnstoffkristall eingeschlossen werden und die mehrfach gewinkelten ungesättigten Fettsäuren im wesentlichen nicht.

Überraschend wurde nun aber gefunden, daß der Harnstoff auch mit mehrfach ungesättigten Fettsäuren, deren Moleküle eine starke Krümmung aufweisen, Einschlußverbindungen bilden kann. Das gilt jedenfalls dann, wenn keine größeren Anteile von Fettsäuren mit geradkettigen oder ziemlich geradkettigen Molekülen vorhanden sind.

Für die oben vorgeschlagene erfindungsgemäße Nutzung dieses Umstands kommen insbesondere omega 3-Fettsäuren in Betracht, die der Körper beim Membranaufbau nutzt. Ferner haben die omega 3-Fettsäuren entzündungshemmende Eigenschaften. Dementsprechend sind sie für medizinische Salben geeignet.

Dabei wird mit der Erfindung ein erheblicher weiterer Fortschritt erzielt. Wie sich ferner gezeigt hat, verlieren aus Fischöl gewonnene Fettsäuren in der Einschlußverbindung mit Harnstoff ihren sonst, wie vor allem von Lebertransalben bekannt, nicht zu verhindernden Geruch. Das ist für die omega 3-Fettsöuren insofern besonders wesentlich, als diese praktisch nur aus Fischöl gewonnen werden können.

Insbesondere in Betracht kommen die Eicosapentaensäure die Docosapentaensäure und die Docosahexaensäure, die sämtlich omega 3-Fettsäuren sind, aber auch die teilweise in der omega 3-Konfiguration auftretende Arachidonsäure und die Octadecatetraensäure.

Mit Fettsäuren" sind im Vorliegenden immer auch ohne ausdrückliche Erwähnung deren genannte Verbindungen gemeint. Als solche kommen hauptsächlich Ethylester in Betracht. Der erfindungsgemäße Bestandteile eines Hautpflegemittels wird sowohl als Vorprodukt zur Herstellung eines Hautpflegemittels als auch als in dem fertigen Hautpflegemittel vorhandener Bestandteil verstanden.

Ein besonders vorteilhafter Aspekt der Erfindung liegt schließlich darin, daß sie eine nützliche Verwendung von Filterrückständen ermöglicht, die bei einer Öltrennung durch Bildung von Einschlußverbindungen mit Harnstoff anfallen.
Im Falle geeigneter Ölmischungen brauchen solche Filterrückstände lediglich, z.B. mit Hexan, ausgewaschen, getrocknet und, vorzugsweise auf unter 50 µm, gemahlen zu werden.

Im folgenden sei die Erfindung anhand von Beispielen weiter erläutert.

Verschiedene Mischungen von Fettsäureethylestern wurden jeweils mit Harnstoff in Ethanol unter Stickstoff-Atmosphäre mit Rückfluß gekocht, bis der Harnstoff sich vollständig gelöst hat. Danach wurde das Gemisch langsam abkühlen gelassen und schließlich nach etwa 6 bis 8 Stunden auf eine Endtemperatur zwischen 5 und 10°C abgekühlt. Der dabei ausgefällte Niederschlag wurde durch Filtrieren abgetrennt, mit Hexan ausgewaschen, getrocknet und gemahlen.
Im folgenden wird, wie auch sonst im Vorliegenden, wieder nur von den Fettsäuren statt von ihren Estern gesprochen.
Es werden jeweils 20 g Öl (Fettsäuremischung) und 40 g Harnstoff wurden in 400 g 100%iges Ethanol gegeben.

### Beispiel 1:

Ausgangsöl war eine die Docosahexaensäure (22:6) und die Docosapentaensäure (22:5), ferner die Heneicosapentaensäure (21:5) sowie einen Teil der Eicosapentaensäure (20:5) enthaltende Fraktion eines Fischöles aus einer chromatographischen Trennung mit überkritischem Kohlendioxid als mobiler Phase verwendet.
In der nachstehenden Tabelle sind die Anteile der Fettsäuren im Ausgangsöl und im Filterrückstand zusammengestellt.

| Fettsäure | Ausgangsöl | Filterrückstand |
|---|---|---|
| 20:5 | 18,6 | 12,52 |
| 21:5 | 7,1 | 7,99 |
| 22:5 | 9,3 | 18,68 |
| 22:6 | 59,7 | 57,06 |

### Beispiel 2:

Als Ausgangsöl wurde eine den Hauptteil der Eicosapentaensäure (20:5), die Arachidonsöure (20:4) omega 3, die Arachindonsäure (20:4) omega 6 sowie weitere Fettsäuren enthaltende Fraktion eines Fischöles aus einer chromatographischen Trennung mit überkritischem Kohlendioxid als mobiler Phase verwendet.
In der nachstehenden Tabelle sind die Anteile der Fettsäuren im Ausgangsöl und im Filterrückstand zusammengestellt.

| Fettsäure | Ausgangsöl | Filterrückstand |
|---|---|---|
| 18:0 | 1,44 | 4,24 |
| 18:1 | 2,07 | 5,37 |
| 18:2 | 0,80 | 2,16 |
| 18:4 | 3,88 | 12,49 |
| 20:4 omega 6 | 2,60 | 2,56 |
| 20:4 omega 3 | 2,84 | 6,08 |
| 20:5 | 80,61 | 54,46 |

### Beispiel 3:

Das Ausgangsöl bestand aus einer mit Eicosapentaensäure (20:5) angereicherten Mischung. In der nachstehenden Tabelle sind die Anteile der Fettsäuren im Ausgangsöl und im Filterrückstand zusammengestellt.

| Fettsäure | Ausgangsöl | Filterrückstand |
|---|---|---|
| 18:1 | 11,72 | 41,54 |
| 18:2 | 3,77 | 14,5 |
| 18:4 | 2,12 | 15,8 |
| 20:4 omega 6 | 1,58 | -- |
| 20:4 omega 3 | 1,77 | 0,96 |
| 20:5 | 47,52 | 6,12 |
| 21:5 | 1,62 | -- |
| 22:5 | 3,52 | -- |
| 22:6 | 11,26 | -- |

Aus einem Vergleich der Beispiele 1 und 2 mit dem Beispiel 3 ist zu erkennen, daß größere Mengen von Fettsäuren mit geradkettigen oder mit, wie die 18:1, ziemlich geradkettigen Molekülen die Einlagerung der mehrfach ungesättigten Fettsäuren behindern und daher vorher möglichst reduziert werden sollten.
Im ganzen läßt sich nach dem beschriebenen Verfahren etwa die Hälfte der omega 3-Fettsäuren eines Gemisches im Harnstoff einschließen.

## Patentansprüche

1. Harnstoff enthaltender Bestandteil eines Hautpflegemittels,
**dadurch gekennzeichnet,**
**daß** der Harnstoff Einschlußverbindungen mit mehrfach ungesättigten Fettsäuren und/oder Verbindungen mehrfach ungesättigter Fettsäuren bildet.

2. Bestandteil nach Anspruch 1,
**dadurch gekennzeichnet,**
**daß** die Verbindungen Fettsäureethylester sind.

3. Bestandteil nach Anspruch 1 oder 2,
**dadurch gekennzeichnet,**
**daß** die Fettsäuren mindestens zum Teil omega 3-Fettsäuren sind.

4. Bestandteil nach einem der Ansprüche 1 bis 3,
**dadurch gekennzeichnet,**
**daß** sich unter den Fettsäuren Octadecatetraensäure, Arachidonsäure, Eicosapentaensäure, Docosapentaensäure und/oder Docosahexaensäure befindet bzw. befinden.

5. Bestandteil nach einem der Ansprüche 1 bis 4,
**dadurch gekennzeichnet,**
**daß** die Einschlußverbindungen in einem bei einer Öltrennung angefallenen Filterrückstand vorliegen.

6. Bestandteil nach Anspruch 5,
**dadurch gekennzeichnet,**
**daß** der Filterrückstand im wesentlichen chemisch unverändert den Bestandteil bildet.

7. Verfahren zum Herstellen eines Bestandteils nach einem der Ansprüche 1 bis 6,
**dadurch gekennzeichnet,**
**daß** ein Öl oder eine aus einem Öl hergestellte Mischung von Fettsäureethylestern mit Harnstoff in einem Lösungsmittel, vorzugsweise Ethanol, unter nichtoxidierender Atmosphäre, vorzugsweise Stickstoff, gekocht wird, vorzugsweise mit Rückfluß, bis der Harnstoff sich gelöst hat, und das Gemisch abkühlen gelassen und schließlich auf eine Endtemperatur unter 10°C gekühlt wird und der dabei ausgefällte Niederschlag, vorzugsweise durch Filteren, abgetrennt wird.

8. Verfahren nach Anspruch 7,
**dadurch gekennzeichnet,**
**daß** der Filterrückstand, vorzugsweise mit Hexan, ausgewaschen, getrocknet und, vorzugsweise auf unter 50 µm, gemahlen wird.

9. Verfahren nach Anspruch 7 oder 8,
**dadurch gekennzeichnet,**
**daß** ein Öl mit geringen Gehalten an Fettsäuren mit geradkettigen oder ziemlich geradkettigen Molekülen verwendet wird.

## Claims

1. A urea-containing component of a skin care agent, **characterized in that** the urea forms inclusion compounds with multiply unsaturated fatty acids and/or compounds of multiply unsaturated fatty acids.

2. The component according to claim 1, **characterized in that** the compounds are fatty acid ethyl esters.

3. The component according to claim 1 or 2, **characterized in that** at least a portion of the fatty acids are omega 3-fatty acids.

4. The component according to any of claims 1 to 3, **characterized in that** the fatty acids are selected from the group consisting of octadecatetreic acid, arachidonic acid, eicosapenteic acid, docosapenteic acid and/or docosahexeic acid.

5. The component according to any of claims 1 to 4, **characterized in that** the inclusion compounds are present in a filter residue obtained from an oil separation.

6. The component according to claim 5, **characterized in that** the filter residue forms the component essentially chemically unchanged.

7. A method of manufacturing a component according to any of claims 1 to 6, **characterized in that** an oil or a mixture of fatty acid ethyl esters manufactured from an oil is cooked, preferably with reflux, with urea in a solvent, preferably ethanol, in a non-oxidizing atmosphere, preferably nitrogen, until the urea has been dissolved, the mixture is permitted to cool, and finally it is cooled to a final temperature of below 10°C and the resulting precipitate is separated, preferably by filtration.

8. The method according to claim 7, the filter residue is washed, preferably with hexane, dried and ground, preferably to below 50 µm.

9. The method according to claim 7 or 8, an oil containing small quantities of fatty acids with straight-chain molecules or fairly straight-chain molecules is used.

## Revendications

1. Constituant d'un produit de soin pour la peau, contenant de l'urée, **caractérisé en ce que** l'urée forme des composés d'inclusion avec des acides gras polyinsaturés et/ou des composés d'acides gras polyinsaturés.

2. Constituant selon la revendication 1, **caractérisé en ce que** les composés sont des esters éthyliques d'acide gras.

3. Constituant selon la revendication 1 ou 2, **caractérisé en ce que** les acides gras sont au moins en partie des acides gras oméga-3.

4. Constituant selon l'une quelconque des revendications 1 à 3, **caractérisé en ce que** parmi les acides gras se trouve(nt) l'acide octadécatétraénoïque, l'acide arachidonique, l'acide eicosapentaénoïque, l'acide docosapentaénoïque et/ou l'acide docosahexaénoïque.

5. Constituant selon l'une quelconque des revendications 1 à 4, **caractérisé en ce que** les composés d'inclusions se trouvent dans un résidu de filtre obtenu lors d'une séparation de l'huile.

6. Constituant selon la revendication 5, **caractérisé en ce que** le résidu de filtre forme le constituant essentiellement non modifié d'un point de vue chimique.

7. Procédé pour la préparation d'un constituant selon l'une quelconque des revendications 1 à 6, **caractérisé en ce qu'**on fait bouillir de préférence à reflux une huile ou un mélange préparé à partir d'huile d'esters éthyliques d'acide gras avec de l'urée dans un solvant, de préférence l'éthanol, sous une atmosphère non oxydante, de préférence nitrogene, jusqu'à la dissolution de l'urée et on laisse refroidir le mélange puis on le refroidit à une température finale inférieure à 10°C et on sépare le précipité formé, de préférence par filtration.

8. Procédé selon la revendication 7, **caractérisé en ce que** le résidu de filtre est lavé, de préférence avec de l'hexane, séché et broyé, de préférence au-dessous de 50 µm.

9. Procédé selon la revendication 7 ou 8, **caractérisé en ce qu'**on utilise une huile présentant des teneurs basses en acides gras présentant des molécules linéaires ou considérablement linéaires.
